# EUROPEAN PATENT APPLICATION

(11) **EP 4 744 493 A1**
(43) Date of publication of application: **20.05.2026**
(21) Application number: 24213032.6
(22) Date of filing: 14.11.2024
(51) Int. Cl.: A22C 17/00, A22C 25/08, A22C 25/12, B65G 47/24, G01N 33/12

(54) **METHOD AND SYSTEM FOR PROCESSING AND/OR INSPECTING A FOOD ITEM**

(71) Applicant: Marel Salmon A/S, 9530 Støvring (DK)
(72) Inventor: Thorsteinsson, Tómas, 9530 Stövring (DK); Genutis, Saulius, 9530 Stövring (DK); Madsen, Preben Kirkegaard, 9530 Stövring (DK); Andersen, Rasmus Frederik Brandt, 9530 Stövring (DK); Jörundsson, Aðalsteinn Rúnar, 9530 Stövring (DK)
(74) Representative: Laddé, Jurre Gerard

(57) **Abstract**

This disclosure relates to a method and system for processing and/or inspecting a food item. The method comprises a leading conveyor conveying the food item in a first direction, towards and onto a trailing conveyor. The leading conveyor and the trailing conveyor are separated by a gap. The method comprises the trailing conveyor conveying the food item in a second direction at least a component of which extends in the first direction. The trailing conveyor conveys the food item faster than the leading conveyor herewith stretching at least a part of the food item when the food item bridges the gap. The method also comprises capturing an image of and/or performing a processing step on the stretched at least part of the food item.

## Description

### FIELD OF THE INVENTION

This disclosure relates to a method and system for processing and/or inspecting a food item, in particular to such method and system wherein a trailing conveyor conveys the food item faster than a leading conveyor. This disclosure further relates to computer-implemented method and a computer for performing the method.

### BACKGROUND

US6407818 discloses a system for scanning for example a fresh meat bulk product. A disadvantage of this system is that it is less suitable for detecting irregularities, such wounds and/or defects and/or signs of sickness, on the surface of such fresh meat bulk product. Hence, there is a need in the art for a system for processing and/or inspecting a food item that at least alleviates this problem.

### SUMMARY

Therefore, one aspect of this disclosure relates to a method for processing and/or inspecting a food item. The method comprises a leading conveyor conveying the food item in a first direction, towards and onto a trailing conveyor. The leading conveyor and the trailing conveyor are separated by a gap. The method comprises the trailing conveyor conveying the food item in a second direction at least a component of which extends in the first direction. The trailing conveyor conveys the food item faster than the leading conveyor herewith stretching at least a part of the food item when the food item bridges the gap. The method also comprises capturing an image of and/or performing a processing step on the stretched at least part of the food item.

One aspect of this disclosure relates to a system for processing and/or inspecting a food item. The system comprises a leading conveyor and a trailing conveyor. The leading conveyor and the trailing conveyor are separated by a gap. Further, the leading conveyor is configured to convey the food item in a first direction, towards and onto the trailing conveyor. The trailing conveyor is configured to convey the food item in a second direction at least a component of which extends in the first direction. Further, the trailing conveyor is configured to convey the food item faster than the leading conveyor for stretching at least a part of the food item when the food item bridges the gap. The system further comprises an imaging system for capturing an image of the stretched at least part of the food item and/or a tool for performing a processing step on the stretched at least part of the food item.

The method and system disclosed herein are advantageous in that they allows to capture images of the food item that clearly show irregularities on the surface of the food item and/or to perform a processing step on the food item more easily and more accurately. The inventors have found that irregularities on a surface of a food item, such as a wound, becomes more clearly visible when the food item is stretched to some extent. One reason would be that the stretching enlarges the irregularity making it more easily detectable. Further, the inventors have found that performing a processing step on the food item easier if the food item is stretched to some extent. To illustrate, it is easier to cut through at least part of a food item if that part is stretched. In the method disclosed herein, the trailing conveyor conveys the food item faster than the leading conveyor, as a result of which the at least part of the food item is stretched when it bridges the gap. Potentially, the at least part of the food item remains stretched even when the food item has been conveyed onto the trailing conveyor completely. In any case, as explained above, any irregularity that would be present in and/or on the stretched at least part would be clearly exposed and would be relatively easy to identify in an image of the stretched part.

A further advantage of the trailing conveyor conveying the food item faster than the leading conveyor is that it may change the orientation of the food item when the food item bridges the gap. To illustrate, the food item may be oriented in a certain direction different from the first direction. The orientation of the food item may for example be the direction from a tail end of the food item towards a front end of the food item. If the food item is a fish, for example, then the orientation of the fish may be the direction from the tail of the fish to the head of the fish. By having the trailing conveyor convey the food item faster than the leading conveyor, the trailing conveyor may cause a pulling effect on the food item in the second direction when the food item bridge the gap, which will cause the orientation of the food item to be aligned more with the second direction. This effect thus causes food items that pass over the gap one after the other to have a more similar orientation when they pass over the gap. Such similar orientation is beneficial as it allows to more effectively detect irregularities and/or to more accurately perform the processing step.

The leading conveyor may comprise a support surface and the trailing conveyor may comprise a support surface as well. The trailing conveyor may convey the food item faster than the leading conveyor because the support surface of the trailing conveyor moves faster than the support surface of the leading conveyor.

At least a component of the second direction extending in the first direction may be understood as that the first and second direction make an angle with each other less than 90 degrees. The angle between the first direction and second direction would be the angle between a first vector representing the first direction and a second vector representing the second direction, wherein the first vector and second vector have the same initial point. Preferably, the angle between the first direction and the second direction is less than 70 degrees, more preferably less than 50 degrees.

The first direction may be understood to be the direction in which the conveyor is conveying the food item when the food item reached the end of the leading conveyor and is conveyed onto the trailing conveyor.

The captured image is preferably a color image to more easily detect irregularities that cause discolorations of the food item.

In an embodiment, capturing the image of and/or performing the processing step on the stretched at least part of the food item is performed when the stretched at least part of the food item is passing over the gap.

This embodiment ensures that the image is captured and/or the processing step is performed when the stretched part is significantly stretched. Note that once the at least stretched part has completely conveyed onto the trailing conveyor, it may relax again, i.e. become less stretched.

In an embodiment, capturing the image of and/or performing the processing step on the stretched at least part of the food item is performed at a first time. The trailing conveyor conveys the food item faster than the leading conveyor herewith stretching at least a second part of the food item when the food item bridges the gap. In this embodiment, the method comprises capturing, at a second time after the first time, a second image of and/or performing a second processing step on the stretched at least second part of the food item, wherein the at least second part of the food time is passing over the gap at the second time.

This embodiment is advantageous as it allows to capture respective images of different stretched parts of the food item thus allowing for a more complete inspection of the food item. Additionally or alternatively, this embodiment allows to perform respective processing steps on different stretched parts of the food item.

The second image is preferably also a color image.

In an embodiment, the method comprises a line scanner system scanning the food item as it passes over the gap herewith capturing the image and the second image.

In an embodiment, the system comprises a line scanner system for scanning the food item as it passes over the gap herewith capturing the image and the second image.

These embodiments provides for an efficient way of obtaining many images of different stretched parts of the food item.

Preferably, the line scanner system captures color images in order to easily detect discolorations of the food item.

A line scanner system is typically configured to capture one so-called image line at a time of an object. Such image line may be understood as a row of image pixels. A line scanner system typically comprises a single row of camera sensor pixels as opposed to a matrix of camera sensor pixels. If the to-be-imaged object moves relative to the line scanner system, and if the line scanner system can capture subsequent image line fast enough, the line scanner system can indeed capture a complete image of the object by capturing one image line at a time while the object is moving relative to the scanner system. All captured image lines may subsequently be combined into a single image. As referred to herein, the image of the stretched at least part of the food item may consists of several image lines as captured by the line scanner system and the second image of the stretched at least second part of the food item may consists of several other image lines as captured by the line scanner system.

The line scanner system would typically be configured to capture an image line of an area in the gap between the leading conveyor and trailing conveyor. In the methods and systems disclosed herein, the line scanner system, in particular its row of camera sensor pixels, preferably has a static position relative to earth.

In an embodiment, the method comprises the leading conveyor conveying the food item in the first direction at a first constant speed, and the trailing conveyor conveying the food item in the second direction at a second constant speed that is higher than the first speed.

This embodiment obviates the need to vary the speed of the leading and trailing conveyor and thus allows for relatively simple control mechanisms.

In an embodiment, the food item is a meat item or a fish item, such as a fish fillet. The method disclosed herein has been found to work especially well for fish fillets, because fish fillets tend to stretch quite well when they bridge the gap between the leading conveyor and the trailing conveyor.

In an embodiment, the method comprises computer-implemented method steps comprising
- determining, based on the captured image, that the food item fails to meet a criterion, and
- based on determining that the food item fails to meet the criterion, controlling a separation system to remove the food item from a main processing line and/or outputting an indication that the food item fails to meet the criterion.

In an embodiment, the system comprises a data processing system configured to perform these computer-implemented steps.

These embodiments are advantageous as it allows to automatically detect and/or separate food items that fail to meet some criterion. An example criterion that may be used is that each food item should not contain an irregularity, or should not contain a specific type of irregularity.

The step of determining that the food item fails to meet the criterion based on the captured image may be performed using a mathematical model that has been constructed by performing a machine-learning method. Such machine-learning method may have involved a neural network known in the art. Constructing the model may for example have been performed by providing training data comprising a plurality of training images, wherein each training image is an image of a similar food item and wherein each training image comprises an image region that represents an irregularity due to which the food item would fail the criterion. The training data would indicate the image region in each training image. A machine-learning method may then construct the mathematical model based on the training data.

In an embodiment, determining that the food item fails to meet the criterion comprises identifying one or more regions in the captured image, each of the one or more regions representing a wound on a skin of the fish fillet.

This embodiment is highly advantageous in that the method disclosed herein work especially well for detecting such wounds on the skin of fish fillets.

In an embodiment, the trailing conveyor conveys the food item 1.01 to 1.07 times faster than the leading conveyor, preferably 1.03 to 1.05 times faster.

The leading conveyor may convey the food item in the first direction at a first speed and the trailing conveyor may convey the food item in the second direction at a second speed, and the trailing conveyor conveying the food item 1.01 to 1.07 times faster than the leading conveyor may be understood as that the ration between the second speed and the first speed is in the range 1.01 to 1.07 (end points included).

The first direction is preferably a downwards direction.

In an embodiment of the method, the food item comprises a first part and a second part, wherein the first part is stiffer than the second part, and wherein the first direction is a downwards direction.

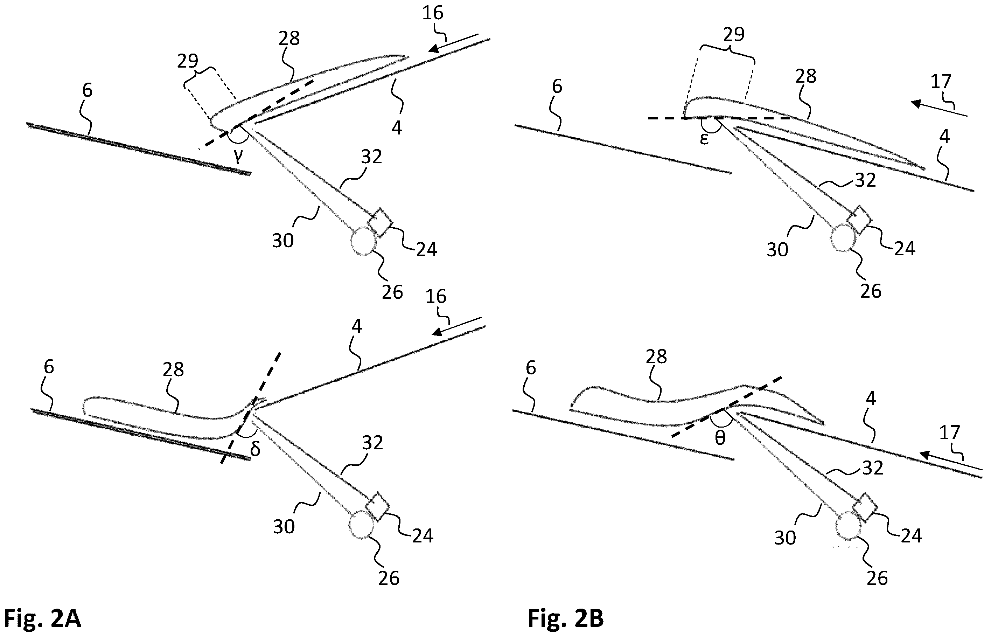

For example, if the food item is a fish item, such as fish filet, then typically the front part of the fish item, which may be understood as the part of the fish item near the head of the fish, is stiffer than the tail part of the fish item, which may be understood as a part of the fish item near the tail of the fish. In such case, it is beneficial if the first direction extends downwards because that ensures that, at any given time while the food item passes over the gap, the part of the food item that sits over or in the gap, remains in a similar orientation. This is beneficial as it improves the quality of the captured images. How this works will be explained in more detail below with reference to figures 2A and 2B.

In an embodiment, the system comprises a data processing system that is configured to control the leading conveyor and the trailing conveyor, and is configured to control the imaging system and/or the tool, wherein the data processing system is configured to cause any of the systems disclosed herein to perform any of the methods disclosed herein.

One aspect of this disclosure relates to a computer-implemented method for controlling any of the systems disclosed herein. The computer-implemented method comprises
- controlling the leading conveyor to convey the food item in the first direction, towards and onto the trailing conveyor, and
- controlling the trailing conveyor to convey the food item in the second direction at least a component of which extends in the first direction, and
- controlling the leading conveyor to convey the food item faster than the leading conveyor for stretching at least a part of the food item when the food item bridges the gap, and
- controlling the imaging system to capture the image of the stretched at least part of the food item and/or the tool to perform the processing step on the stretched at least part of the food item.

Controlling any of the leading conveyor, trailing conveyor, imaging system, tool may be performed by sending appropriate control signals to respectively the leading conveyor, trailing conveyor, imaging system, tool.

One aspect of this disclosure relates to a computer program comprising instructions which, when the program is executed by the any of the data processing systems referred to herein, cause any of the systems disclosed herein to perform any of the methods disclosed herein.

For example, the computer program may comprise instructions which, when the program is executed by the data processing system, cause the data processing system to perform the computer-implemented method for controlling the system herewith causing the system to perform any of the methods disclosed herein.

One aspect of this disclosure relates to a data processing system comprising a computer readable storage medium having computer readable program code embodied therewith, and a processor, preferably a microprocessor, coupled to the computer readable storage medium, wherein responsive to executing the computer readable program code, the processor is configured to perform any of the computer-implemented methods disclosed herein.

A data processing system may also be referred to as a computer.

One aspect of this disclosure relates to a computer-readable medium having stored thereon any of the computer programs disclosed herein.

One aspect of this disclosure relates to a non-transitory computer-readable storage medium storing at least one software code portion, the software code portion, when executed or processed by a computer, is configured to perform any of the computer-implemented methods disclosed herein.

One aspect of this disclosure relates to a computer-readable data carrier having stored thereon any of the computer programs described herein. The computer-readable data carrier may be hard disk, for example, or a signal.

One aspect of this disclosure relates to a data carrier signal carrying any of the computer programs described herein.

A distinct aspect of this disclosure relates to a method inspecting a food item. In this distinct aspect, the method comprises a leading conveyor conveying the food item in a first direction, towards and onto a trailing conveyor, wherein the first direction is a downwards direction. The leading conveyor and the trailing conveyor are separated by a gap. The method comprises the trailing conveyor conveying the food item in a second direction at least a component of which extends in the first direction. The method also comprises capturing a first image of a first part of the food item as the first part passes the gap and capturing a second image of a second part of the food item as the second part passes the gap.

A further distinct aspect of this disclosure relates to a system for inspecting a food item. The system comprises a leading conveyor and a trailing conveyor. The leading conveyor and the trailing conveyor are separated by a gap. Further, the leading conveyor is configured to convey the food item in a first direction, towards and onto the trailing conveyor, wherein the first direction is a downwards direction. The trailing conveyor is configured to convey the food item in a second direction at least a component of which extends in the first direction. The system further comprises an imaging system for capturing a first image of a first part of the food item as the first part passes the gap and for capturing a second image of a second part of the food item as the second part passes the gap.

In these last two distinct aspects, the trailing conveyor need not necessarily convey the food item faster than the leading conveyor. The method and system as respectively defined in the foregoing two paragraphs are especially advantageous if the food item has a first part, such as a front part of the food item, that is stiffer than a second part, such as a rear part of the food item, because the method and system of the foregoing two paragraphs ensure that, at any given time while the food item passes over the gap, the part that is passing the gap remains in a same orientation relative to the imaging system. How this works will be explained in more detail with reference to figures 2A and 2B.

Elements and aspects discussed for or in relation with a particular embodiment may be suitably combined with elements and aspects of other embodiments, unless explicitly stated otherwise. Embodiments of the present invention will be further illustrated with reference to the attached drawings, which schematically will show embodiments according to the invention. It will be understood that the present invention is not in any way restricted to these specific embodiments.

### BRIEF DESCRIPTION OF THE DRAWINGS

Aspects of the invention will be explained in greater detail by reference to exemplary embodiments shown in the drawings, in which:
FIG. 1A is a perspective view of a system for processing and/or inspecting a food item according to an embodiment;
FIG. 1B is a side view of the system of figure 1A;
FIG. 2A shows side views of a system according to an embodiment in operation wherein the leading conveyer moves the food item in a downwards direction;
FIG. 2B shows side views of a system according to an embodiment in operation wherein the leading conveyer moves the food item in an upwards direction;
FIG. 3 illustrates a data processing system according to an embodiment.

### DETAILED DESCRIPTION OF THE DRAWINGS

In the figures, identical reference numbers indicate identical or similar elements.

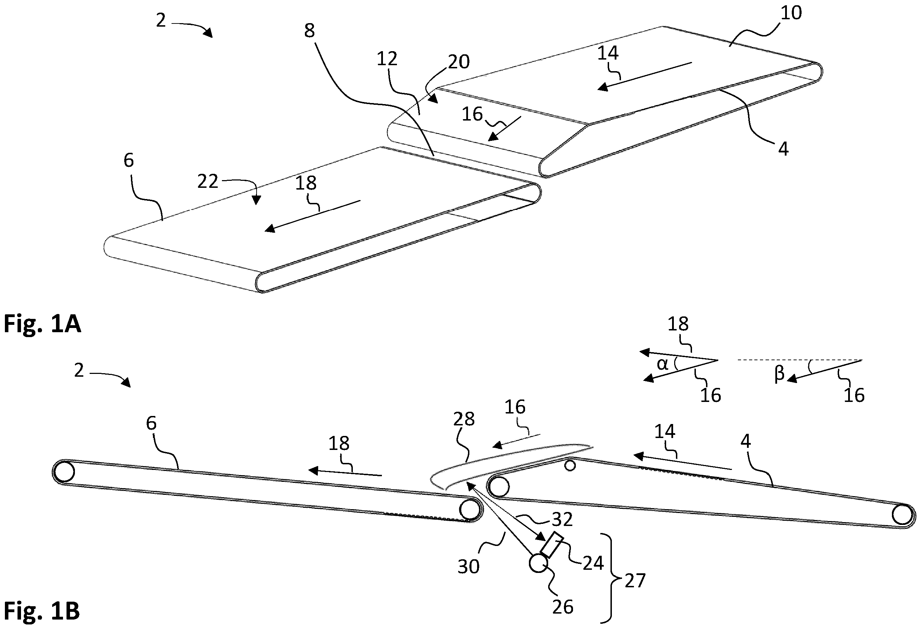

Figures 1A and 1B illustrates a system 2 for processing and/or inspecting a food item. Figure 1A shows a perspective view and figure 1B a side view. The system 2 comprises a leading conveyor 4 and a trailing conveyor 6, which are separated by a gap 8. In the depicted embodiment, the gap 8 is a slit between the leading conveyor 4 and trailing conveyor 6. The leading conveyor 4 comprises a support surface 20 that, in operation, moves while supporting a food item 28, which is for example a fish or fish fillet. Likewise, the trailing conveyor 6 comprises a support surface 22 that, in operation moves while supporting food item 28. The conveyors 4 and 6 are typically belt conveyors and the support surfaces 20 and 22 are surfaces of the respective belts of conveyors 4 and 6. Typically, the conveyors would transport the food item at a constant speed. The leading conveyor 4 is configured to convey the food item 28 in a slight upwards direction indicated by arrow 14 at a first part 10 of the leading conveyor 4, but the last part 12 of leading conveyor 4 is configured to convey the food item 28 in a direction indicated by arrow 16, which is a slightly downwards direction. Direction 16 makes an angle β with the horizontal direction as indicates. Angle β may for example be between 5 and 45 degrees, preferably between 10 and 25 degrees. The leading conveyor 4 is configured to convey the food item 28 onto the trailing conveyor 6 as can be seen in figure 1B. Then, the trailing conveyor 6 conveys the food item 28 in a direction indicated by arrow 18. The angle α between direction 16 and direction 18 is smaller than 90 degrees. Preferably, angle α is smaller than 45 degrees. In operation, the trailing conveyor 6 conveys the food item 28 faster than the leading conveyor 4 so that at least a part of the food item 28 is stretched when the food item 28 bridges the gap 8, for example 1.01 to 1.07 times faster. A further effect of the trailing conveyor 6 conveying the food item 28 faster than the leading conveyor is that it may straighten the food item 28 to some extent in that it may cause the orientation of the food item to be more aligned with direction 18 already when it passes over the gap 8. This aids to ensure that every food item that passes over the gap 8 has more or less the same orientation, which may ease image analysis and detection of irregularities in captured images.

The system further comprises an imaging system 27 for capturing an image of the stretched at least part of the food item 28 and/or a tool for performing a processing step on the stretched at least part of the food item 28. In the depicted embodiment, the imaging system 27 is configured to capture an image of a bottom of the food item 28. To facility this, the imaging system 27 is positioned lower than the leading conveyor 4. The imaging system 27 shows comprises a light source 26 that is configured to shine light 30 through the gap 8 on the part of the food item 28 that passes over the gap 8. At least part of this light 30 reflects from the food item 28 and the reflected light 32 reaches an imaging device 24 of the imaging system 27, in particular an imaging plane of imaging device 24 so that an image is captured of the stretched part of the food item 28. The imaging system 27 is preferably a line-scan system that is able to capture color images. The resulting image may be analyzed by an AI model, and the result of this analysis will be available for reporting and decision-making with regards to routing of the food item 28.

The stretching of the food item improves the quality of the images that are taken by the imaging system 27 in the sense that the stretching may expose and/or enlarge irregularities on the food item 28, especially on a surface of the food item 28. The system is especially suitable for detecting exterior defects on the skin side of fish fillets, because fish fillets tend to be relatively easily stretchable. To illustrate, the system can effectively scan salmon fillets to detect winter wounds. If the system for example detects winter wounds on a salmon fillet, then this fillet may be separated from the main line for close inspection.

Figures 2A and 2B illustrate the benefit of having the leading conveyor 4 convey the food item 28 in a downwards direction 16, especially if a front part 29 of the food item 28 is stiffer than a rear part of the food item 28. Figure 2A shows an embodiment of the system wherein the leading conveyor 4 indeed transports the food item 28 in a downwards direction, at least when the food item is transferred onto trailing conveyor 6, whereas figure 2B shows an embodiment of the system wherein the leading conveyor 4 moves the food item in an upwards direction 17.

In figure 2A (top), the food item has reached the gap between leading conveyor 4 and trailing conveyor 6. A front part of the food item 28, which is for example the part of a fish near the head, already extends beyond the leading conveyor 4. Especially if this front part 29 is relatively stiff, relative to for example a rear part of the food item, then the front part 29 will bend downwards only to a limited extent and the front part 29 will, to some extent, remain oriented along direction 16 even though it extends beyond conveyor 4. As can be seen in figure 2A (top), the light 30 from the light source 26 hits the food item roughly at an angle γ. Angle γ is approximately 105 degrees in figure 2A.

In figure 2A (bottom), the rear part of the food item 28 passes over the gap. The rear part, especially if it is less stiff than the head part 29, will bend downwards stronger than the head part 29. As a result, the light 30 is incident on the food item 28 at an angle δ that is smaller than angle γ. Angle δ is approximately 75 degrees in figure 2A. The difference between angles γ and δ in figure 2A is relatively small compared to the embodiment of figure 2B as will be explained now.

In figure 2B, the leading conveyer 4 moves the food item 28 in an upwards direction 17. In figure 2B (top), the food item 28 has reached the gap 8 and the front part 29 already extends beyond the leading conveyor 4. Especially if the front part 29 of the food item is stiffer than a rear part of the food item, will the front part 29 that extends beyond the leading conveyor 4 bend downwards only to a limited extent. The front part 29 will thus remain oriented roughly along direction 17. As a result, the light 30 of light source 26 will hit the food item roughly at a relatively large angle ε. Angle ε is approximately 45 degrees in figure 2B.

In figure 2B (bottom), the rear part of food item 28 passes over the gap. The rear part, especially if it is less stiff than the front part 29, will bend downwards to a greater extent than the front part 29. As a result, the light 30 is incident on the food item roughly at an angle θ. Angle θ is approximately 110 degrees in figure 2B. The difference between angle ε and angle θ is thus relatively large. Such varying angle between light 30 and the food item 28 while the food item is passing over the gap is undesired, as it means that the orientation of the food item 28 varies relative to the imaging system (irrespective of where the imaging is positioned). It is readily understood that such varying angle complicates capturing of comparable images. One reason may for example be that the intensity of reflected light 32 varies greatly due to the varying orientation of the food item 28. To illustrate, in figure 2B (top), only a small fraction of the incident light 30 will be reflected towards the imaging device 24, whereas in figure 2B (bottom) a significantly larger fraction of the incident light 30 will be reflected towards imaging device 24 due to angle θ being closer to 90 degrees than angle ε. Such varying intensity 32 that is incident on camera 24 deteriorates the quality of the captured image.

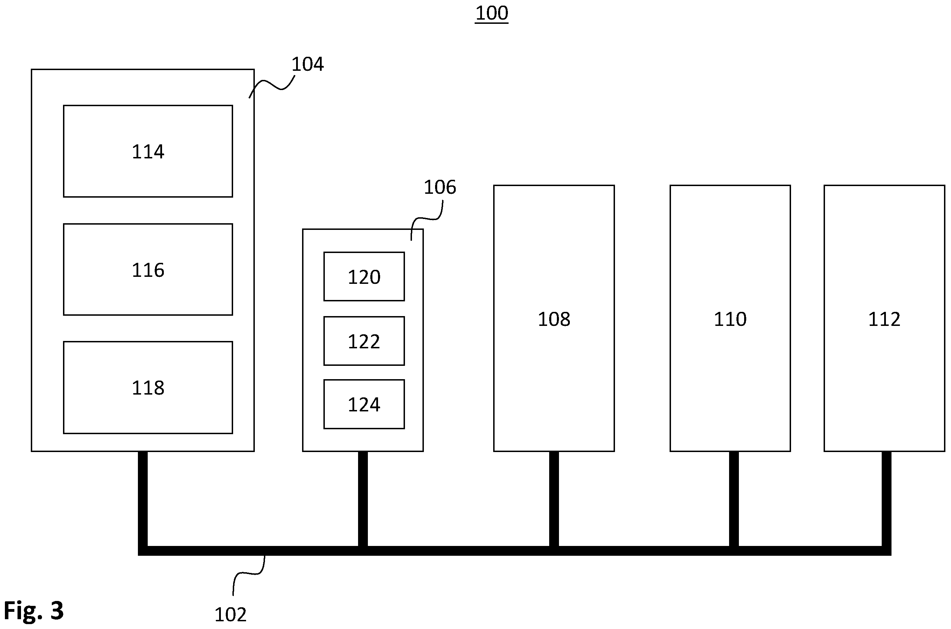

Figure 3 schematically illustrates a data processing 100, also referred to as a computer, according to an embodiment. The data processing system 100 may for example represent a controller as described herein.

Data processing system 100 may be configured to control the leading conveyor and the trailing conveyor, and to control the imaging system and/or the tool referred to herein. The data processing system 100 is configured to cause any of the systems disclosed herein to perform any of the methods disclosed herein.

The data processing system 100 is configured to, in an embodiment, perform a method comprising determining, based on the captured image, that the food item fails to meet a criterion, and based on determining that the food item fails to meet the criterion, controlling a separation system to remove the food item from a main processing line and/or outputting an indication that the food item fails to meet the criterion. The data processing system 100 for example determines the food item fails to meet a criterion by identifying one or more regions in the captured image, each of the one or more regions representing a wound on a skin of the fish fillet or representing any other irregularity on the fish fillet.

The data processing system 100 is preferably configured to perform steps of
- controlling the leading conveyor to convey the food item in the first direction, towards and onto the trailing conveyor, and
- controlling the trailing conveyor to convey the food item in the second direction at least a component of which extends in the first direction, and
- controlling the leading conveyor to convey the food item faster than the leading conveyor for stretching at least a part of the food item when the food item bridges the gap, and
- controlling the imaging system to capture the image of the stretched at least part of the food item and/or the tool to perform the processing step on the stretched at least part of the food item.

The data processing system 100 may control these elements by sending appropriate control signals to these element.

In the depicted data processing system 100, a system bus 102 connects the different components of the data processing system 100. In particular, the system bus 102 depicted in figure 3 connects the Central Processing Unit (CPU) 104, memory elements 106, input devices 108, output devices 110 and communication devices 112 with each other so that they can exchange information. The system bus 102 may be understood to serve both as data bus, address bus and control bus known in the art.

The CPU 104 is configured to perform steps as per the instructions comprised in a computer program. To illustrate, based on such instructions, the CPU may perform any of the computer-implemented methods described herein. Typically, the CPU 104 is embodied as a microprocessor, which can be implemented on a single metal-oxide-semiconductor integrated circuit chip. The CPU 104 comprises a control unit 114, an arithmetic logical unit (ALU) 116 and a plurality of registers 118.

The control unit 114 is configured to retrieve instructions from a main memory 120. Typically, the control unit 114 comprises a binary decoder to convert the retrieved instructions into timing and control signals that direct the operation of for example the ALU 116. ALU 116 is configured to perform logical operations, such as additions, subtraction, multiplication, division and Boolean operations, that are required for carrying out the instructions. The registers 118 are small memory elements that can be read and written at relatively high speed. A register may for example store an instruction, a storage address, or any other kind of data. In addition, the CPU may contain hardware caches known in the art (not shown). Preferably, the CPU has different levels of caches. These hardware caches may be understood as an intermediate state between the faster registers 119 and the slower main memory 120.

Memory elements 106 comprise a main memory 120. The main memory 120, also referred to as primary storage in the art, has stored data that is directly accessible to the CPU 104. The CPU 104 may continuously read instructions, i.e. read computer programs, stored in the main memory 120 and execute these instructions. The main memory 120 is typically a random access memory (RAM).

Memory elements 106 further comprise so-called secondary storage 122, which may be embodied as one or more hard disk drives and/or as one or more solid state drives. Typically, this secondary storage is non-volatile. Further, the memory elements may comprise other storage devices 124, such as removable storage devices, e.g. CD, DVD, USB flash drives, floppy disks, et cetera.

Input devices 108 may be understood as devices that are used to provide information to the computer 100, in particular to the CPU 104. Non-limiting examples of input devices are a keyboard, a microphone, a joystick, a mouse, a touch sensitive screen, et cetera. Also sensors that are configured to for example detect when a food item passes over a gap are examples of input devices. Further, the data processing system may receive data from an imaging system referred to herein, which data represent a captured image of the stretched part of the food item. In that sense, the imaging system may also be regarded as an input device to the data processing system 100.

Output devices 110 may be understood as devices that output information out of the computer and/or as devices that are controlled by the computer. Non-limiting examples of output devices 110 are a display, a printer, a headphones, loudspeaker, the leading conveyor, the trailing conveyor, in particular motors that drive these conveyors, the imaging system referred to herein, in particular the light source and imaging device of the imaging system, et cetera.

Communication devices 112 may be understood as devices that allow the computer system to communicate with other computers, such as with a server computer, client computer, or any other type of remote device. Non-limiting examples of communication devices 112 include modems, cable modems, ethernet cards, Bluetooth modules, et cetera.

## Claims

1. A method for processing and/or inspecting a food item, the method comprising
a leading conveyor conveying the food item in a first direction, towards and onto a trailing conveyor, wherein
the leading conveyor and the trailing conveyor are separated by a gap, the method comprising
the trailing conveyor conveying the food item in a second direction at least a component of which extends in the first direction, wherein
the trailing conveyor conveys the food item faster than the leading conveyor herewith stretching at least a part of the food item when the food item bridges the gap, and the method comprises
capturing an image of and/or performing a processing step on the stretched at least part of the food item.

2. The method according to claim 1, wherein capturing the image of and/or performing the processing step on the stretched at least part of the food item is performed when the stretched at least part of the food item is passing over the gap.

3. The method according to claim 2, wherein
capturing the image of and/or performing the processing step on the stretched at least part of the food item is performed at a first time, and wherein
the trailing conveyor conveys the food item faster than the leading conveyor herewith stretching at least a second part of the food item when the food item bridges the gap, further comprising
capturing, at a second time after the first time, a second image of and/or performing a second processing step on the stretched at least second part of the food item, wherein the at least second part of the food time is passing over the gap at the second time.

4. The method according to claim 3, comprising a line scanner system scanning the food item as it passes over the gap herewith capturing the image and the second image.

5. The method according to any one of the preceding claims, further comprising the leading conveyor conveying the food item in the first direction at a first constant speed, and the trailing conveyor conveying the food item in the second direction at a second constant speed that is higher than the first speed.

6. The method according to any one of the preceding claims, wherein the food item is a meat item or a fish item, such as a fish fillet.

7. The method according to any one of the preceding claims, further comprising computer-implemented method steps of
- determining, based on the captured image, that the food item fails to meet a criterion, and
- based on determining that the food item fails to meet the criterion, controlling a separation system to remove the food item from a main processing line and/or outputting an indication that the food item fails to meet the criterion.

8. The method according to claims 6 and 7, wherein determining that the food item fails to meet the criterion comprises identifying one or more regions in the captured image, each of the one or more regions representing a wound on a skin of the fish fillet.

9. The method according to any one of the preceding claims, wherein the trailing conveyor conveys the food item 1.01 to 1.07 times faster than the leading conveyor, preferably 1.03 to 1.05 times faster.

10. The method according to any one of the preceding claims, wherein
the food item comprises a first part and a second part, wherein the first part is stiffer than the second part, and wherein
the first direction is a downwards direction.

11. A system for processing and/or inspecting a food item, the system comprising a leading conveyor, and
a trailing conveyor, wherein
the leading conveyor and the trailing conveyor are separated by a gap, wherein
the leading conveyor is configured to convey the food item in a first direction, towards and onto the trailing conveyor, wherein
the trailing conveyor is configured to convey the food item in a second direction at least a component of which extends in the first direction, wherein
the trailing conveyor is configured to convey the food item faster than the leading conveyor for stretching at least a part of the food item when the food item bridges the gap, and
an imaging system for capturing an image of the stretched at least part of the food item and/or a tool for performing a processing step on the stretched at least part of the food item.

12. The system according to claim 11, further comprising
a data processing system that is configured to control the leading conveyor and the trailing conveyor, and is configured to control the imaging system and/or the tool, wherein the data processing system is configured to cause the system of claim 11 to perform the method according to any of claims 1-10.

13. The system according to claim 11 or 12, wherein the first direction is a downwards direction.

14. A computer-implemented method for controlling the system according to any of claims 11 - 13, comprising
- controlling the leading conveyor to convey the food item in the first direction, towards and onto the trailing conveyor, and
- controlling the trailing conveyor to convey the food item in the second direction at least a component of which extends in the first direction, and
- controlling the leading conveyor to convey the food item faster than the leading conveyor for stretching at least a part of the food item when the food item bridges the gap, and
- controlling the imaging system to capture the image of the stretched at least part of the food item and/or the tool to perform the processing step on the stretched at least part of the food item.

15. A computer program comprising instructions which, when the program is executed by the data processing system referred to in claim 12, cause the system of claim 12 to perform the method according to any one of claims 1-10.
